# EUROPEAN PATENT APPLICATION

(11) **EP 1 045 248 A2**
(43) Date of publication of application: **18.10.2000**
(21) Application number: 00107951.6
(22) Date of filing: 17.04.2000
(51) Int. Cl.: G01N 33/543, G01N 33/58

(54) **Luminescent labeled immunoassay method and analysis element therefor**

(30) Priority: 16.04.1999 JP 10906899
(71) Applicant: FUJI PHOTO FILM CO., LTD., Kanagawa 250-01 (JP)
(72) Inventor: Shinoki, Hiroshi, c/o Fuji Photo Film Co., Ltd., Saitama (JP); Seshimoto, Osamu, c/o Fuji Photo Film Co., Ltd., Saitama (JP)
(74) Representative: Albrecht, Thomas, Dr.

(57) **Abstract**

A liquid sample containing an analyte antibody and a solution containing an enzyme-labeled second antibody are fed successively to a microporous membrane on which an antigen is immobilized, to form a complex of the immobilized antigen, the antibody and the enzyme-labeled second antibody. Thereafter, an enzyme substrate is fed to the membrane to cause a luminous reaction on the membrane. The existence and the quantity of the antibody are detected based on the luminous quantity. The use of chemiluminescence enables to detect the antibody at extremely high sensitivity. When the luminescence is detected by means of a photography film, adjustment of the exposure time enables to set up the sensitivity at any magnitude. Especially, use of an instant photography film can provide an extremely convenient and rapid detection of the antibody.

## Description

### FIELD OF THE INVENTION

The present invention relates to a luminescent or luminometric enzyme immunoassay and an analysis element therefor. Particularly, the present invention relates to a membrane immunoassay where a luminescent or luminometric immunoassay is applied, and to an assaying method capable of detecting conveniently and rapidly a specific antibody in a liquid sample and an analysis element therefor.

### BACKGROUND OF THE INVENTION

An immunoassay has been widely used as a rapid and sensitive assaying method for determining the existence or the quantity of a substance (generally, referred to as "analyte") present at a low concentration in a liquid sample. The immunoassay utilizes a specific binding reaction between an antigen (or hapten) and an antibody and is particularly useful since it provides a high level of specificity and sensitivity.

Recently, among the immunoassay, an enzyme immunoassay (EIA) in which either of an antibody or an antigen is labeled with an enzyme is widely used owing to its capability of detecting an analyte quantitatively at a high sensitivity. However, the conventional EIA is not fully a convenient method since it requires for several hours for judgment and uses a special equipment at washing operation for B/F separation.

For example, in diagnosis at an early stage of infection like the case of examination of hepatitis, it is necessary to detect rapidly the existence of an antibody against a specific virus in a number of test samples. Hepatitis is an infectious disease where an immunoassay based on the detection of an anti-virus antibody is useful. Such assay is useful not only as a tool for pure diagnosis but also for screening before blood transfusion and screening blood donators. Hepatitis B and C or serum hepatitis has been observed worldwide, especially at drug abusers among which the disease is generally infectious through a syringe needle for subcutaneous injection commonly used.

As convenient immunoassays for examination of hepatitis, a particle agglutination test (PA test) using gelatin particles sensitized by an antigen and a hemagglutination assay using erythrocytes are known, but they take about 2 hours for judgment and thus lack rapidness. Furthermore, they have a disadvantage that judgment of the existence of agglutination is difficult and requires skill.

Assaying methods which are relatively convenient and require no special equipment include a membrane immunoassay described in Unexamined Japanese Patent Publication No. H03-206961(1991) (corresponding to EP 0420170A). The method comprises immobilizing an antigen onto a microporous membrane such as a nitrocellulose membrane, adding thereto a liquid sample containing an analyte antibody to cause a binding reaction between the antigen and the antibody on the membrane, and then adding a solution containing an enzyme-labeled second antibody, followed by adding a substrate solution to produce a colored product through an enzymatic reaction after an excessive enzyme-labeled antibody is removed by washing. Thus, the presence of the analyte antibody is determined by visually detecting the presence of colored spot(s) on the membrane. This method characteristically utilizes a capillary action of the microporous membrane to realize passages of the liquid sample and the second antibody solution, and also successive washing operation through the antigen-immobilized microporous membrane. To promote the liquid flow by capillary action, an absorbing pad is placed under the microporous membrane. This method is often referred to as a flow-through assay (U.S. Patent Nos. 3,888,629, 4,246,339, 4,632,901, 4,277,560 and 4,812,293).

Although the membrane EIA is convenient to require no special equipment, the sensitivity is not satisfactory. Furthermore, it is often necessary to read the developed color immediately after the assay since the developed color on the membrane changes with the passage of time in some test sample cases. In afore-mentioned Unexamined Japanese Patent Publication No. H03-206961(1981) (which corresponds EP 0420170A), after development of the color, a stabilizing solution is passed through the microporous membrane in order to stabilize the developed color. Therefore, it is impossible to finish the assay within a short period of time owing to increased steps for the assay.

### SUMMARY OF THE INVENTION

The present invention has been accomplished in consideration of the above situation, and a first object thereof is to provide an assaying method for detecting an antibody conveniently and rapidly at high sensitivity. The second object of the present invention is to provide an analysis element suitable for the assaying method.

The first object of the present invention is achieved by a luminescent labeled immunoassay method for analyzing the existence or the quantity of an antibody in a liquid sample, comprising the steps of:
(a) contacting the liquid sample with a microporous membrane on which an antigen is immobilized, to form an antigen-antibody complex on the microporous membrane;
(b) supplying, to the microporous membrane, an enzyme-labeled second antibody which is a conjugate of a labeling enzyme and a second antibody being capable of binding to the antibody;
(c) removing, from the microporous membrane, the excessive enzyme-labeled second antibody which has not bound to the antigen-antibody complex;
(d) applying a substrate to the membrane to cause a luminous reaction between the labeling enzyme and the substrate; and
(e) detecting or measuring the luminescence produced to determine the existence or the quantity of the antibody in the sample.

According to the present invention, a liquid sample containing an analyte antibody and an enzyme-labeled second antibody are fed successively to a microporous membrane on which an antigen corresponding to the analyte antibody is immobilized, to form a complex of three members, i.e., the immobilized antigen, the analyte antibody and the enzyme-labeled second antibody. Then, an enzyme substrate is applied to the membrane to cause a luminous reaction on the membrane. The existence of the target analyte antibody in the sample can be judged by detecting the existence of luminescence on the membrane. Furthermore, measurement of the intensity of the luminescence on the membrane enables to determine the quantity and concentration of the analyte antibody in the sample.

The existence of luminescence and the luminous quantity can be detected by means of a photosensitive film such as a photography film, a luminometer, or a CCD camera. However, use of an instant photography employing an instant photography film is particularly suitable as a convenient and inexpensive method.

The use of a photography film enables to enhance sensitivity of the luminous quantity, i.e., sensitivity of the detection of the analyte antibody, by lengthening the exposure quantity (time). Accordingly, the present invention has a great advantage for qualitative or semi-quantitative assay where a possibility of a disease is judged positive or negative based on the presence or absence of an antibody at a level of not less than a certain cut-off value.

In the assay method of the present invention, when a liquid sample is spotted onto a microporous membrane containing an immobilized antigen placed on a water-absorbing layer, an antigen-antibody reaction takes place and an aimed antibody (immunoglobulin) is captured on the membrane. The other immunoglobulins which do not participate in the antigen-antibody reaction migrate to the water-absorbing layer through washing in a perpendicular direction, i.e., a direction across the membrane. Next, when an enzyme-labeled antibody (a second antibody) which is capable of binding to the aimed antibody is added thereto, the enzyme-labeled antibody is captured on the membrane. For removing the excessive enzyme-labeled antibody, washing is conducted again utilizing the water-absorbing layer. Then, an enzyme substrate is added to the membrane to make it luminous for detection.

The second object of the present invention is achieved by an analysis element for a luminescent labeled immunoassay to analyze the existence or the quantity of an antibody in a liquid sample through a reaction of an antigen with an enzyme-labeled second antibody against the antibody, which comprises:
a microporous membrane on which an antigen against said antibody is immobilized; and
a water-absorbing layer arranged under the microporous membrane, which absorbs a liquid fed to the microporous membrane.

The element wherein the water-absorbing layer is detachable from the microporous membrane is one preferable embodiment.

More preferably, the water-absorbing layer may contain the substance for inhibiting a luminous reaction by the label enzyme. In this case, it is possible to prevent occurrence of the luminous reaction without detaching the water absorptive layer from the microporous membrane.

### BRIEF DESCRIPTION OF THE DRAWINGS

Fig. 1 is an illustration showing the principal layer construction of an assay element according to the present invention and schematic representations of the concept of the assay method thereof;
Fig. 2 is a photographic image taken through an instant photography film showing the results of Example 3;
Fig. 3 is a photographic image taken through an instant photography film showing the results of Example 6; and
Fig. 4 is a photographic image taken through an instant photography figure showing the results of the comparative example in Example 6.

### DESCRIPTION OF THE PREFERRED EMBODIMENTS

### Analyte (Substance to Be Analyzed)

The substance to be analyzed by the present invention is an antibody contained in an aqueous liquid sample. Any antibody can be the substance to be analyzed provided that an antigen can specifically bind to the (aimed or target) antibody is obtainable. Examples of such antibody include antibodies against an antigen of hepatitis C virus (HCV), an surface antigen of Hepatitis B virus (HBs), HIV, syphilis, chlamydia, tubercle bacillus, and the like. However, the substance to be analyzed by the present invention is not limited thereto.

### Layer Construction of Analysis Element and Principle of the Invention

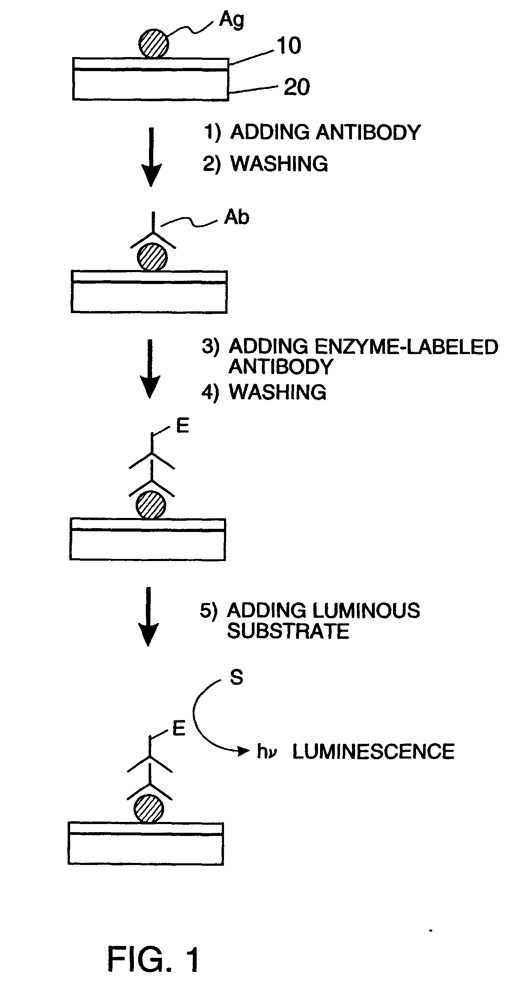

In Fig. 1, the reference numeral 10 represents a microporous membrane, which is superposed on a water-absorbing layer 20 having a sufficient water-absorbing action or capacity. On the microporous membrane, an antigen (Ag) which can be specifically bound with an analyte antibody is immobilized in advance. When a sample solution containing the analyte antibody is applied or spotted on the microporous membrane 10 containing the immobilized antigen, an antigen-antibody binding reaction takes place on the membrane and an aimed antibody (Ab: an immunoglobulin) is trapped on the membrane (step 1). In the case that the liquid sample or specimen e.g., blood, body fluid, and the like, contains immunoglobulin fractions other than the aimed analyte antibody, it is desirable that the other immunoglobulins are transferred to the water absorptive layer 20, together with the liquid components of the sample, in a perpendicular direction, i.e., a direction across the membrane, by applying a washing liquid of a suitable buffer solution to the membrane 10, to remove them from the membrane 10 (step 2).

Next, when an enzyme-labeled antibody (Ab-E: a second antibody) which reacts with the aimed analyte antibody is added thereto, the enzyme-labeled antibody is trapped on the membrane (step 3). For removing the excessive enzyme-labeled antibody, washing liquid is fed again to the membrane 10 to wash the membrane with utilizing the water-absorbing layer (step 4). Then, a luminous substrate (S) which causes a luminescence through a reaction with the labeling enzyme is added. The existence of the luminescence (hν) or the luminous quantity is detected (step 5). In the case that the luminous quantity is detected by means of a photography film, the microporous membrane to which the luminous substrate has been added is placed in a dark box and the film is exposed.

### Microporous Membrane

The microporous membrane on which an antigen is immobilized may be anyone capable of immobilizing the antigen densely. For example, microporous membranes made of materials such as nitrocellulose, (charge-improved) nylon, and vinylidene fluoride resin (PVDF).

Immobilization of the antigen onto the microporous membrane may be achieved by a covalent bonding with a chemical reaction or by a physical adsorption. After the antigen is immobilized, it is preferable to block the membrane surface with BSA and the like in order to prevent non-specific adsorption of an antibody at assay. These are conducted according to a hitherto known method.

### Water-Absorbing Layer

The water-absorbing layer 20 is placed under the immobilized antigen layer 10 in order to promote a capillary action of the microporous membrane 10 and achieve absorption and removal of water or liquid efficiently. The absorptive layer 20 is made of an water absorptive material including a porous fibrous material such as cellulose, cellulose acetate, rayon, cotton, or glass fiber; or gelatin, polysaccharide, a polymer having pyrrolidone skeleton, poly(methyl vinyl ether), a highly water-absorptive polymer, or the like. The water absorbing layer 20 may have a water-absorbing capacity sufficient to absorb the sample solution, the second antibody solution, the washing liquid and the like added to the upper-layered microporous membrane and to pass them through the membrane.

### Enzyme-Labeled Antibody (Second Antibody)

The enzyme-labeled antibody (the second antibody) may be any antibody that specifically recognizes and binds to the analyte antibody, and may be a monoclonal antibody or a polyclonal antibody. In the case that the analyte antibody is a human antibody, a rabbit, a goat, or sheep is immunized with a human immunoglobulin fraction and an antibody against human immunoglobulin obtained after purification may be used as the second antibody. Recently, anti-Fc antibody that specifically recognizes Fc region of human IgG is available from several reagent makers. The antibody can be labeled with an enzyme according to a known method.

The labeling enzyme may be selected from enzymes capable of reacting with the substrates forming luminous substances through reactions with the enzymes. Examples of the labeling enzyme include alkaline phosphatase (ALP), horse radish peroxidase (HRP), β-galactosidase (β-GAL), and luciferase. The substrate forming a luminous substance may be selected based on the labeling enzyme. For example, when ALP is used as a label enzyme, CSPD (trade name of Boehringer Mannheim Co., Ltd.; 3-(4-methoxyspiro[1,2-dioxetane-3,2'(5'-chloro)-tricyclo[3.3.1.1^{3,7}]decan]-4-yl)phenyl phosphate) can be used as a luminous substrate. For HRP, luminol/enhancer may be used as a luminous substrate. For luciferase, luciferin/ATP is used as a luminous substrate. These can be selected from combinations of enzymes and luminous substrates widely known as chemiluminescence detecting methods.

### Substance for Inhibiting Luminous Reaction

The excessive enzyme-labeled antibody, which has not bound to the antigen-antibody complex on the microporous membrane 10, migrates into the water-absorbing layer 20. When a part of the luminous substrate fed to the porous membrane 10 migrates into the water-absorbing layer 20, the free enzyme-labeled antibody causes a luminous reaction also in the water-absorbing layer 20. The luminous reaction in the lower water-absorbing layer produces a noise to increase a background for measurement. Such a undesirable reaction can be prevented by, after the unreacted enzyme-labeled antibody is washed (step 4 in Fig. 1), detaching the water-absorbing layer 20 from the microporous membrane 10 and then carrying out a luminous reaction through adding a luminous substrate solution to the membrane (cf. Example 3 described hereinafter).

Alternatively, a substance for inhibiting a luminous reaction may be included in the water-absorbing layer 20. The luminous reaction can be thereby carried out through adding a luminous substrate without detaching the water-absorbing layer 20 from the microporous membrane 10, as described in Example 6, in details, hereinafter.

The luminous reaction inhibitor may be any one preventing a reaction of a free enzyme-labeled antibody with a luminous substrate in the water-absorbing layer 20. For example, an inhibitor for the label enzyme may be employed. An antibody specific to an active site of the enzyme may also be used as the substance for inhibiting a luminous reaction. Alternatively, a protease such as pepsin may be included in the water-absorbing layer 20 and the labeling enzyme of the free enzyme-labeled antibody transferred into the layer 20 through washing operation is thereby decomposed and inactivated. Thus, such a protease may be used as the inhibitor. The substance decomposing or inactivating a coenzyme of the label enzyme may be also used as the luminous reaction inhibitor.

An enzyme inhibitor against alkaline phosphatase includes a chelating agent such as EDTA that traps zinc metal existing at the active center of the enzyme. In the case of a peroxidase, sodium azide can be employed as an enzyme inhibitor.

### Luminometry

The luminous quantity may be determined by detecting with a luminometer, a CCD camera, and an instant photography film. Among them, an instant photography film is particularly suitable as a convenient and inexpensive detecting method.

### Example 1

### Preparation of HCV Antigen-Immobilized Membrane

Equal amounts of each 100 µg/mL solution (10 mM phosphate buffer solution, pH 7.2) of HCV core, HCVNS3, and HCVNS4 (ABI Co., Ltd.) were mixed and the resulting solution was spotted on a nitrocellulose membrane (SNHF) made by Millipore Corporation in an amount of 5 µL (at each position of the membrane). The membrane was then dried at room temperature for 30 minutes under a reduced pressure. The nitrocellulose membrane was immersed in 10 mM phosphate buffer solution (pH 7.2) containing 1% BSA (SIGMA Co., Ltd.) to subject to a blocking at room temperature for 4 hours. Then, the membrane was dried at room temperature one overnight under a reduced pressure.

### Example 2

### Preparation of HCV Antigen-Immobilized Membrane Slide -

The antigen-immobilized nitrocellulose membrane prepared in Example 1 was cut into pieces having sizes of about 15 mm x 15 mm so that the position at which the antigen had been spotted located in the center of each piece. A plastic sheet of the size of 2.4 cm x 2.8 cm having an opening of 1 cm diameter was adhered to the surface of the membrane as a cover of the slide so that the antigen-spotted position located in the center of opening of the sheet. Four sheets of a commercially available cosmetic cotton puff was piled up and fixed with an adhesive tape into a compressed state. The four-layered puff was adhered to the back of the nitrocellulose membrane as an absorptive material (water-absorbing layer).

### Example 3

### Measurement of HCV Antibody

A positive control serum against HCV antibody was diluted with 10 mM phosphate buffer (pH 7.2) into 4 fold, 16-fold, 64-fold, and 256-fold dilutions. These dilution series of serum were assayed. A negative control serum to HCV antibody was used as a reference.

Each of the reagent solutions was dropped and absorbed to the opening of cover of the slide prepared in Example 2 in the order described below.
1) 10 µL of Human serum of positive control against HCV antibody
2) 120 µL of 10 mM phosphate buffer solution (pH 7.2)
3) 60 µL of ALP labeled anti-human IgG goat antibody (TROPIX Co., Ltd.)
4) 360 µL of 10 mM phosphate buffer solution (pH 7.2)

Next, the absorptive material (water-absorbing layer) was detached from the slide to make it the one made of the cover and the nitrocellulose membrane only. A mixture of 10 µL of CSPD (trademark: Boehringer Mannheim Co., Ltd) in a reagent kit "BM Chemiluminescence ELISA Substrate (AP)" made by Boehringer Mannheim Co., Ltd, 100 µL of an enhancer solution, and 890 µL of an assay buffer was used as an ALP substrate solution for chemiluminescence. The ALP substrate solution was loaded on the opening of the cover in an amount of 300 µL each. The slide was then set in a dark box for detecting luminescence and the detection was conducted by means of an instant monochrome film (Photorama FP-3000B: made by Fuji Photo Film Co., Ltd.). The exposure time was 20 seconds. Furthermore, each slide from which the absorptive material was removed was placed on each flat bottom well of six-wells microplate and the plate was set on a luminometer (made by TEKAN) to measure a luminous quantity (RLU: Relative Light Unit).

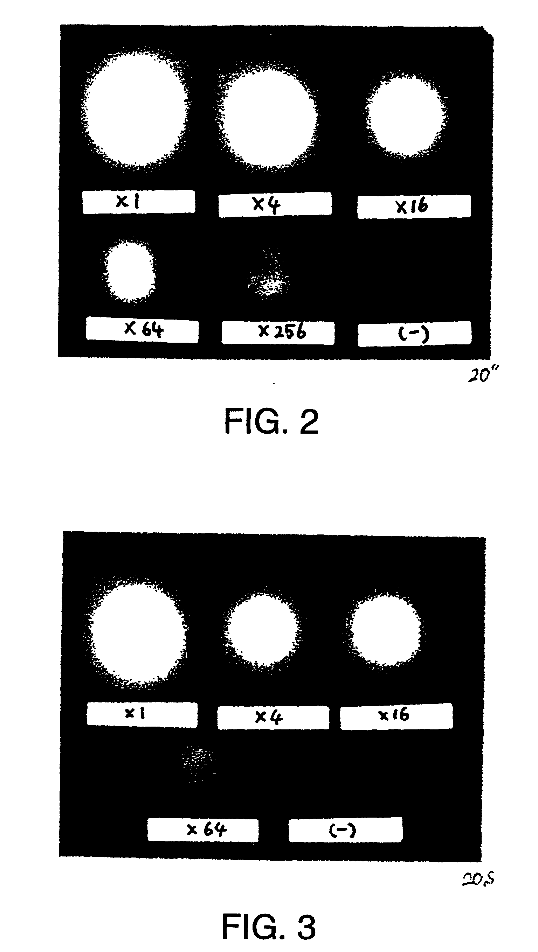

As shown in an instant photography image of Fig. 2, the luminous quantity decreased, depending on the concentration, as dilution fold increased from (x1) to (x256) but even at a low concentration of 256-fold dilution, the existence of HCV antibody could be detected. However, luminescence could not be detected in the case of a comparative example, i.e., the serum of negative control (-). This fact shows complete removal of background. Accordingly, it is expectable that a lower concentration of HCV antibody can be detected when the exposure time is lengthened.

The luminous quantity detected by means of the luminometer increased depending on the concentration of the test antibody in the liquid sample as shown in the following Table 1. This fact shows capability of quantitative assay of the analyte antibody by the luminescent labeled immunoassay according to the present invention.

**Table 1**

| The Results of Measurement on Luminometer | |
|---|---|
| Dilution Fold | RLU (Relative Light Unit) |
| 1 | 12590 |
| 4 | 3312 |
| 16 | 915 |
| 64 | 183 |
| 256 | 56 |
| Negative serum (control) | 0 |

### Example 4

### Preparation of HBs Antigen-Immobilized Membrane

HBs antigen was dissolved in 10 mM phosphate buffer solution (pH 7.2) to be a 100 µg/mL solution. The resulting solution was spotted on a nitrocellulose membrane (SNHF) made by Millipore Corporation in an amount of 5 µL (at each position of the membrane). The membrane was then dried at room temperature for 30 minutes under a reduced pressure. The nitrocellulose membrane was Immersed in 10 mM phosphate buffer solution (pH 7.2) containing 1% BSA (SIGMA Co., Ltd.) to subject to a blocking at room temperature for 4 hours. Then, the membrane was dried at room temperature one overnight under a reduced pressure.

### Example 5

### Preparation of HBs Antigen-Immobilized Membrane Slide

The antigen-immobilized nitrocellulose membrane prepared in Example 4 was cut into pieces having sizes of about 15 mm x 15 mm so that the position at which the antigen has been spotted located in the center of each piece. A plastic sheet of the size of 2.4 cm x 2.8 cm having an opening of 1 cm diameter was adhered to the surface of the membrane as a cover of the slide so that the antigen-spotted position located in the center of opening of the sheet. A commercially available cosmetic cotton puff was immersed in 2 mg/mL EDTA-2Na salt solution (10 mM phosphate buffer solution) for 10 minutes and then dried at room temperature under reduced pressure. The puff was cut into pieces of a size of about 2.5 cm x 3 cm. Four pieces of the cut puff was piled up and fixed with an adhesive tape into a compressed state. The four-layered puff was adhered to the back of the nitrocellulose membrane as an absorptive material (water-absorbing layer).

### Example 6

### Measurement of HBs Antibody

A positive control serum of HBs antibody was diluted with 10 mM phosphate buffer (pH 7.2) into 4-fold, 16-fold and 64-fold dilutions. The dilution series of the serum were assayed. A negative control serum of HBs antibody was used as a reference.

Each of the reagent solutions was dropped and absorbed to the opening of cover of the slide prepared in Example 5 at the order described below.
1) 10 µL of Human serum of positive control against HBS antibody
2) 120 µL of 10 mM phosphate buffer solution (pH 7.2)
3) 60 µL of ALP labeled anti-human IgG goat antibody (TROPIX Co., Ltd.)
4) 360 µL of 10 mM phosphate buffer solution (pH 7.2)

The same substrate solution as used in Example 3 was used as an ALP substrate solution for chemiluminescence. The substrate solution was loaded on the opening of the cover of each slide in an amount of 300 µL each. The slide was then set in a dark box for detecting luminescence and the detection was conducted by means of an instant monochrome film (photorama FP-3000B: made by Fuji Photo Film Co., Ltd.). The piled cotton puffs were not removed from the slide in this operation. The exposure time was 20 seconds. The total time for the above measuring operation is about 2 minutes.

As a comparative example, measurement of HBs antibody was conducted in a similar manner to Example 6 using a slide prepared as described in Examples 4 and 5 with the exception that a cotton puff not impregnated with EDTA-2Na was used.

As shown in an instant photography image of Fig. 3, a luminous quantity depending on the dilution fold from (x1) to (x64) was observed without detaching the absorptive material (water-absorbing layer) at the luminous reaction in Example 6. Even at a low concentration of 64-fold dilution, the existence of HBs antibody could be detected. However, luminescence could not be detected in the case of the serum of negative control (-). This fact shows complete removal of background. Accordingly, it is expectable that a lower concentration of HBs antibody can be also detected when the exposure time is lengthened.

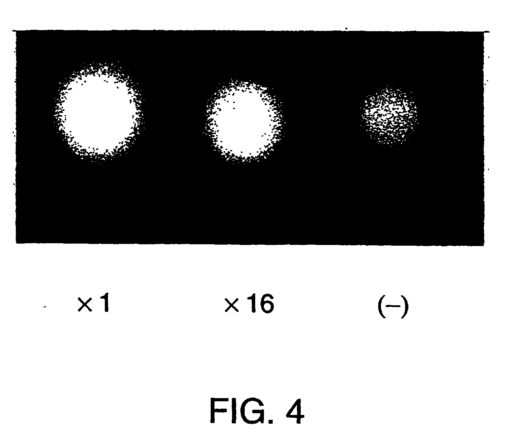

As shown in an instant photography image of Fig. 4, a high luminous quantity was detected even at the serum of negative control (-) in the comparative example where a cotton puff not impregnated with EDTA-2Na was used as the absorptive material (absorbing layer) and the material was not removed the slide at the luminous reaction. These facts show that excess amount of the enzyme-labeled antibody migrated to the adsorptive material (water-absorbing layer) by washing process and reacted with the enzyme substrate therein, resulting in a high background value. Clearly, the method according to the comparative example is not suitable for detecting lower concentration of an antibody owing to the existence of such background.

On the contrary, the background value was low enough to be almost negligible in Example 6 where an absorptive material impregnated with EDTA-2Na, which is an inhibitor to ALP (alkaline phosphatase), was used. Therefore, there is no fear of judgment of pseudo-positive, even when the sensitivity is enhanced by lengthening the exposure time.

As described above, according to the present invention, a liquid sample containing an analyte antibody and a solution containing an enzyme-labeled second antibody are fed successively to a microporous membrane on which an antigen corresponding to the analyte antibody is immobilized, to form a complex of the immobilized antigen, the antibody and the enzyme-labeled second antibody. Thereafter, an enzyme substrate is fed to the membrane to cause a luminous reaction on the membrane. The use of chemiluminescence enables to detect the antibody at extremely high sensitivity. Furthermore, when the luminescence is detected by means of a photography film, change of the exposure time enables to set up the sensitivity at any magnitude. Especially, use of an instant photography film can provide an extremely convenient and rapid detection of the antibody.

Moreover, the background value can be lowered to an almost negligible level by including a substance for inhibiting a luminous reaction such as an enzyme inhibitor in a water-absorbing layer. Accordingly, no possibility of judgment of pseudo-positive exists even when the sensitivity is enhanced by lengthening the exposure time.

## Claims

1. A luminescent labeled immunoassay method for analyzing the existence or the quantity of an antibody in a liquid sample, comprising the steps of;
(a) contacting the liquid sample with a microporous membrane on which an antigen is immobilized, to form an antigen-antibody complex on the microporous membrane;
(b) supplying, to the microporous membrane, an enzyme-labeled second antibody which is a conjugate of a labeling enzyme and a second antibody being capable of binding to the antibody;
(c) removing, from the microporous membrane, the excessive enzyme-labeled second antibody which has not bound to the antigen-antibody complex;
(d) applying a substrate to the membrane to cause a luminous reaction between the labeling enzyme and the substrate; and
(e) detecting or measuring the luminescence produced to determine the existence or the quantity of the antibody in the sample.

2. The luminescent labeled immunoassay according to claim 1, wherein the intensity of the luminescence is determined by a photosensitive film.

3. The luminescent labeled immunoassay according to claim 2, wherein said photosensitive film is an instant photography film.

4. The luminescent labeled immunoassay according to claim 1, wherein after the step (a), the microporous membrane is washed to remove immunoglobulin other than the antibody to be analyzed.

5. The luminescent labeled immunoassay according to claim 1, wherein said microporous membrane is superposed on a water-adsorbing layer.

6. The luminescent labeled immunoassay according to claim 5, wherein said water-absorbing layer contains a substance for inhibiting said luminous reaction.

7. The luminescent labeled immunoassay according to claim 1, wherein said labeling enzyme is any one of peroxidase, β-galactosidase, alkaline phosphatase, and luciferase.

8. The luminescent labeled immunoassay according to claim 1, wherein said second antibody is an anti-Fc antibody.

9. An analysis element for a luminescent labeled immunoassay to analyze the existence or the quantity of an antibody in a liquid sample through a reaction of an antigen with an enzyme-labeled second antibody against the antibody, which comprises:
a microporous membrane on which an antigen against said antibody is immobilized; and
a water-absorbing layer arranged under the microporous membrane, which absorbs a liquid fed to the microporous membrane.

10. The analysis element according to claim 9, wherein the water-absorbing layer is detachable from the microporous membrane.

11. The analysis element according to claim 9, wherein the water-absorbing layer contains a substance for inhibiting a luminous reaction with the labeling enzyme.
